# EUROPEAN PATENT APPLICATION

(11) **EP 3 421 037 A1**
(43) Date of publication of application: **02.01.2019**
(21) Application number: 17178451.5
(22) Date of filing: 28.06.2017
(51) Int. Cl.: A61K 31/454, A61K 31/7105, A61K 31/713, A61P 25/00, G01N 33/68

(54) **RLS TREATMENT WITH INHIBITORS OF THE CEREBLON (CRBN) PROTEIN**

(71) Applicant: Klinikum rechts der Isar der Technischen Universität München, 81675 München (DE)
(72) Inventor: WINKELMANN, Juliane, 80539 München (DE)
(74) Representative: Vossius & Partner Patentanwälte Rechtsanwälte mbB

(57) **Abstract**

The present invention relates to an inhibitor of the cereblon (CRBN) protein for use in the prevention and/or treatment of restless leg syndrome (RLS) and/or diseases associated therewith. The invention also relates to a method of preventing and/or treating restless leg syndrome (RLS) and/or diseases associated therewith comprising administering a pharmaceutically effective amount of an inhibitor of the cereblon (CRBN) protein to a subject in need thereof. Finally, the invention also relates to a method of identifying an inhibitor of the cereblon (CRBN) protein suitable as a lead compound and/or as a medicament for the prevention and/or treatment of restless leg syndrome (RLS) and/or diseases associated therewith.

SEQUENCE LISTING

<110> Klinikum rechts der Isar der Technischen Universität München

<120> RLS treatment with inhibitors of the cereblon (CRBN) protein

<130> AA1093 EP/A

<160> 6

<170> BiSSAP 1.3.6

<210> 1
<211> 442
<212> PRT
<213> Homo sapiens


<220>
<223> cereblon, isoform 1

<400> 1

<210> 2
<211> 441
<212> PRT
<213> Homo sapiens

<220>
<223> cereblon, isoform 2

<400> 2
Met Ala Gly Glu Gly Asp Gln Gln Asp Ala Ala His Asn Met Gly Asn
1               5                   10                  15
His Leu Pro Leu Leu Pro Glu Ser Glu Glu Glu Asp Glu Met Glu Val
            20                  25                  30
Glu Asp Gln Asp Ser Lys Glu Ala Lys Lys Pro Asn Ile Ile Asn Phe
        35                  40                  45
Asp Thr Ser Leu Pro Thr Ser His Thr Tyr Leu Gly Ala Asp Met Glu
    50                  55                  60
Glu Phe His Gly Arg Thr Leu His Asp Asp Asp Ser Cys Gln Val Ile
65                  70                  75                  80
Pro Val Leu Pro Gln Val Met Met Ile Leu Ile Pro Gly Gln Thr Leu
                85                  90                  95
Pro Leu Gln Leu Phe His Pro Gln Glu Val Ser Met Val Arg Asn Leu
            100                 105                 110
Ile Gln Lys Asp Arg Thr Phe Ala Val Leu Ala Tyr Ser Asn Val Gln
        115                 120                 125
Glu Arg Glu Ala Gln Phe Gly Thr Thr Ala Glu Ile Tyr Ala Tyr Arg
    130                 135                 140
Glu Glu Gln Asp Phe Gly Ile Glu Ile Val Lys Val Lys Ala Ile Gly
145                 150                 155                 160
Arg Gln Arg Phe Lys Val Leu Glu Leu Arg Thr Gln Ser Asp Gly Ile
                165                 170                 175
Gln Gln Ala Lys Val Gln Ile Leu Pro Glu Cys Val Leu Pro Ser Thr
            180                 185                 190
Met Ser Ala Val Gln Leu Glu Ser Leu Asn Lys Cys Gln Ile Phe Pro
        195                 200                 205
Ser Lys Pro Val Ser Arg Glu Asp Gln Cys Ser Tyr Lys Trp Trp Gln
    210                 215                 220
Lys Tyr Gln Lys Arg Lys Phe His Cys Ala Asn Leu Thr Ser Trp Pro
225                 230                 235                 240
Arg Trp Leu Tyr Ser Leu Tyr Asp Ala Glu Thr Leu Met Asp Arg Ile
                245                 250                 255
Lys Lys Gln Leu Arg Glu Trp Asp Glu Asn Leu Lys Asp Asp Ser Leu
            260                 265                 270
Pro Ser Asn Pro Ile Asp Phe Ser Tyr Arg Val Ala Ala Cys Leu Pro
        275                 280                 285
Ile Asp Asp Val Leu Arg Ile Gln Leu Leu Lys Ile Gly Ser Ala Ile

290                 295                 300
Gln Arg Leu Arg Cys Glu Leu Asp Ile Met Asn Lys Cys Thr Ser Leu
305                 310                 315                 320
Cys Cys Lys Gln Cys Gln Glu Thr Glu Ile Thr Thr Lys Asn Glu Ile
                325                 330                 335
Phe Ser Leu Ser Leu Cys Gly Pro Met Ala Ala Tyr Val Asn Pro His
            340                 345                 350
Gly Tyr Val His Glu Thr Leu Thr Val Tyr Lys Ala Cys Asn Leu Asn
        355                 360                 365
Leu Ile Gly Arg Pro Ser Thr Glu His Ser Trp Phe Pro Gly Tyr Ala
    370                 375                 380
Trp Thr Val Ala Gln Cys Lys Ile Cys Ala Ser His Ile Gly Trp Lys
385                 390                 395                 400
Phe Thr Ala Thr Lys Lys Asp Met Ser Pro Gln Lys Phe Trp Gly Leu
                405                 410                 415
Thr Arg Ser Ala Leu Leu Pro Thr Ile Pro Asp Thr Glu Asp Glu Ile
            420                 425                 430
Ser Pro Asp Lys Val Ile Leu Cys Leu
        435                 440

<210> 3
<211> 215
<212> PRT
<213> Homo sapiens


<220>
<223> cereblon, variant

<400> 3
Met Glu Glu Phe His Gly Arg Thr Leu His Asp Asp Asp Ser Cys Gln
1               5                   10                  15
Val Ile Pro Val Leu Pro Gln Val Met Met Ile Leu Ile Pro Gly Gln
            20                  25                  30
Thr Leu Pro Leu Gln Leu Phe His Pro Gln Glu Val Ser Met Val Arg
        35                  40                  45
Asn Leu Ile Gln Lys Asp Arg Thr Phe Ala Val Leu Ala Tyr Ser Asn
    50                  55                  60
Val Gln Glu Arg Glu Ala Gln Phe Gly Thr Thr Ala Glu Ile Tyr Ala
65                  70                  75                  80
Tyr Arg Glu Glu Gln Asp Phe Gly Ile Glu Ile Val Lys Val Lys Ala
                85                  90                  95
Ile Gly Arg Gln Arg Phe Lys Val Leu Glu Leu Arg Thr Gln Ser Asp
            100                 105                 110
Gly Ile Gln Gln Ala Lys Val Gln Ile Leu Pro Glu Cys Val Leu Pro
        115                 120                 125
Ser Thr Met Ser Ala Val Gln Leu Glu Ser Leu Asn Lys Cys Gln Ile
    130                 135                 140
Phe Pro Ser Lys Pro Val Ser Arg Glu Asp Gln Cys Ser Tyr Lys Trp
145                 150                 155                 160
Trp Gln Lys Tyr Gln Lys Arg Lys Phe His Cys Ala Asn Leu Thr Ser
                165                 170                 175
Trp Pro Arg Trp Leu Tyr Ser Leu Tyr Asp Ala Glu Thr Leu Met Asp
            180                 185                 190
Arg Ile Lys Lys Gln Leu Arg Glu Trp Asp Glu Asn Leu Lys Asp Asp
        195                 200                 205
Ser Leu Pro Ser Asn Pro Ile
    210                 215

<210> 4
<211> 2596
<212> DNA
<213> Homo sapiens

<220>
<223> cereblon, isoform 1

<400> 4
tgtcccagcc tcctttgcgg gtaaacagac atggccggcg aaggagatca gcaggacgct 60

gcgcacaaca tgggcaacca cctgccgctc ctgcctgcag agagtgagga agaagatgaa 120

atggaagttg aagaccagga tagtaaagaa gccaaaaaac caaacatcat aaattttgac 180

accagtctgc cgacatcaca tacataccta ggtgctgata tggaagaatt tcatggcagg 240

actttgcacg atgacgacag ctgtcaggtg attccagttc ttccacaagt gatgatgatc 300

ctgattcccg gacagacatt acctcttcag ctttttcacc ctcaagaagt cagtatggtg 360

cggaatttaa ttcagaaaga tagaaccttt gctgttcttg catacagcaa tgtacaggaa 420

agggaagcac agtttggaac aacagcagag atatatgcct atcgagaaga acaggatttt 480

ggaattgaga tagtgaaagt gaaagcaatt ggaagacaaa ggttcaaagt ccttgagcta 540

agaacacagt cagatggaat ccagcaagct aaagtgcaaa ttcttcccga atgtgtgttg 600

ccttcaacca tgtctgcagt tcaattagaa tccctcaata agtgccagat atttccttca 660

aaacctgtct caagagaaga ccaatgttca tataaatggt ggcagaaata ccagaagaga 720

aagtttcatt gtgcaaatct aacttcatgg cctcgctggc tgtattcctt atatgatgct 780

gagaccttaa tggacagaat caagaaacag ctacgtgaat gggatgaaaa tctaaaagat 840

gattctcttc cttcaaatcc aatagatttt tcttacagag tagctgcttg tcttcctatt 900

gatgatgtat tgagaattca gctccttaaa attggcagtg ctatccagcg acttcgctgt 960

gaattagaca ttatgaataa atgtacttcc ctttgctgta aacaatgtca agaaacagaa 1020

ataacaacca aaaatgaaat attcagttta tccttatgtg ggccgatggc agcttatgtg 1080

aatcctcatg gatatgtgca tgagacactt actgtgtata aggcttgcaa cttgaatctg 1140

ataggccggc cttctacaga acacagctgg tttcctgggt atgcctggac tgttgcccag 1200

tgtaagatct gtgcaagcca tattggatgg aagtttacgg ccaccaaaaa agacatgtca 1260

cctcaaaaat tttggggctt aacgcgatct gctctgttgc ccacgatccc agacactgaa 1320

gatgaaataa gtccagacaa agtaatactt tgcttgtaaa cagatgtgat agagataaag 1380

ttatctaaca aattggttat attctaagat ctgctttgga aattattgcc tctgatacat 1440

acctaagtaa acataacatt aatacctaag taaacataac attacttgga gggttgcagt 1500

ttctaagtga aactgtattt gaaactttta agtatacttt aggaaacaag catgaacggc 1560

agtctagaat accagaaaca tctacttggg tagcttggtg ccattatcct gtggaatctg 1620

atatgtctgg tagcatgtca ttgatgggac atgaagacat ctttggaaat gatgagatta 1680

tttcctgtgt taaaaaaaaa aaaaatctta aattcctaca atgtgaaact gaaactaata 1740

atttgatcct gatgtatggg acagcgtatc tgtaccagtg ctctaaataa caaaagctag 1800

ggtgacaagt acatgttcct tttggaaaga agcaaggcaa tgtatattaa ttattctaaa 1860

agggctttgt tcctttccat tttctttaac ttctctgaga tactgatttg taaattttga 1920

aaattagtta aaatatgcag ttttttgagc ccacgaatag ttgtcatttc ctttatgtgc 1980

ctgttagtaa aaagtagtat tgtgtatttg ctcagtatct gaactataag cccatttata 2040

ctgttccata caaaagctat ttttcaaaaa ttaatttgaa ccaaaactac tactataggg 2100

aaaagatgcc aaaacatgtc ccctcaccca ggctaaactt gatactgtat tattttgttc 2160

aatgtaaatt gaagaaaatc tgtaagtaag taaaccttaa gtgtgaaact aaacatgttc 2220

tttgttcaaa taatgtaaaa tatctactca taattttaaa gttctaaaaa ggctgctttg 2280

ccacctacct ctttgcttat attcaagggt ttagtgggtc cttccttcca catttgtatg 2340

ataactttcg ttttattggt agtcctttgc tactttaaaa ctaatcaggt taaatgttta 2400

ctcaaatgta gtaatagtat gaggccaaaa tacatgcctt gaaggtggag tgaaatcagt 2460

tgagtgtcac actgcatatt tatgtaaata gagtaaatga aaattcaaga atacttccaa 2520

tcaggatatg cccctgcctt attttttcta aggtctactt ttactacttt aataattttg 2580

ggtttgtgtg aaaaaa 2596

<210> 5
<211> 2593
<212> DNA
<213> Homo sapiens

<220>
<223> cereblon, isoform 2

<400> 5
tgtcccagcc tcctttgcgg gtaaacagac atggccggcg aaggagatca gcaggacgct 60

gcgcacaaca tgggcaacca cctgccgctc ctgcctgaga gtgaggaaga agatgaaatg 120

gaagttgaag accaggatag taaagaagcc aaaaaaccaa acatcataaa ttttgacacc 180

agtctgccga catcacatac atacctaggt gctgatatgg aagaatttca tggcaggact 240

ttgcacgatg acgacagctg tcaggtgatt ccagttcttc cacaagtgat gatgatcctg 300

attcccggac agacattacc tcttcagctt tttcaccctc aagaagtcag tatggtgcgg 360

aatttaattc agaaagatag aacctttgct gttcttgcat acagcaatgt acaggaaagg 420

gaagcacagt ttggaacaac agcagagata tatgcctatc gagaagaaca ggattttgga 480

attgagatag tgaaagtgaa agcaattgga agacaaaggt tcaaagtcct tgagctaaga 540

acacagtcag atggaatcca gcaagctaaa gtgcaaattc ttcccgaatg tgtgttgcct 600

tcaaccatgt ctgcagttca attagaatcc ctcaataagt gccagatatt tccttcaaaa 660

cctgtctcaa gagaagacca atgttcatat aaatggtggc agaaatacca gaagagaaag 720
tttcattgtg caaatctaac ttcatggcct cgctggctgt attccttata tgatgctgag 780
accttaatgg acagaatcaa gaaacagcta cgtgaatggg atgaaaatct aaaagatgat 840
tctcttcctt caaatccaat agatttttct tacagagtag ctgcttgtct tcctattgat 900
gatgtattga gaattcagct ccttaaaatt ggcagtgcta tccagcgact tcgctgtgaa 960
ttagacatta tgaataaatg tacttccctt tgctgtaaac aatgtcaaga aacagaaata 1020
acaaccaaaa atgaaatatt cagtttatcc ttatgtgggc cgatggcagc ttatgtgaat 1080
cctcatggat atgtgcatga gacacttact gtgtataagg cttgcaactt gaatctgata 1140
ggccggcctt ctacagaaca cagctggttt cctgggtatg cctggactgt tgcccagtgt 1200
aagatctgtg caagccatat tggatggaag tttacggcca ccaaaaaaga catgtcacct 1260
caaaaatttt ggggcttaac gcgatctgct ctgttgccca cgatcccaga cactgaagat 1320
gaaataagtc cagacaaagt aatactttgc ttgtaaacag atgtgataga gataaagtta 1380
tctaacaaat tggttatatt ctaagatctg ctttggaaat tattgcctct gatacatacc 1440
taagtaaaca taacattaat acctaagtaa acataacatt acttggaggg ttgcagtttc 1500
taagtgaaac tgtatttgaa acttttaagt atactttagg aaacaagcat gaacggcagt 1560
ctagaatacc agaaacatct acttgggtag cttggtgcca ttatcctgtg gaatctgata 1620
tgtctggtag catgtcattg atgggacatg aagacatctt tggaaatgat gagattattt 1680
cctgtgttaa aaaaaaaaaa aatcttaaat tcctacaatg tgaaactgaa actaataatt 1740
tgatcctgat gtatggacag cgtatctgtt accagtgctc taaataacaa aagctagggt 1800
gacaagtaca tgttcctttt ggaaagaagc aaggcaatgt atattaatta ttctaaaagg 1860
gctttgttcc tttccatttt ctttaacttc tctgagatac tgatttgtaa attttgaaaa 1920
ttagttaaaa tatgcagttt tttgagccca cgaatagttg tcatttcctt tatgtgcctg 1980
ttagtaaaaa gtagtattgt gtatttgctc agtatctgaa ctataagccc atttatactg 2040
ttccatacaa aagctatttt tcaaaaatta atttgaacca aaactactac tatagggaaa 2100
agatgccaaa acatgtcccc tcacccaggc taaacttgat actgtattat tttgttcaat 2160
gtaaattgaa gaaaatctgt aagtaagtaa accttaagtg tgaaactaaa catgttcttt 2220
gttcaaataa tgtaaaatat ctactcataa ttttaaagtt ctaaaaaggc tgctttgcca 2280
cctacctctt tgcttatatt caagggttta gtgggtcctt ccttccacat ttgtatgata 2340
actttcgttt tattggtagt cctttgctac tttaaaacta atcaggttaa atgtttactc 2400
aaatgtagta atagtatgag gccaaaatac atgccttgaa ggtggagtga aatcagttga 2460
gtgtcacact gcatatttat gtaaatagag taaatgaaaa ttcaagaata cttccaatca 2520

ggatatgccc ctgccttatt ttttctaagg tctactttta ctactttaat aattttgggt 2580

ttgtgtgaaa aaa 2593

<210> 6
<211> 758
<212> DNA
<213> Homo sapiens

<220>
<223> cereblon, variant

<400> 6
tgtcccagcc tcctttgcgg gtaaacagac atggccggcg aaggagatca gcaggacgct 60

gcgcacaaca tgggcaacca cctgccgctc ctgcctgtac ctaggtgctg atatggaaga 120

atttcatggc aggactttgc acgatgacga cagctgtcag gtgattccag ttcttccaca 180

agtgatgatg atcctgattc ccggacagac attacctctt cagctttttc accctcaaga 240

agtcagtatg gtgcggaatt taattcagaa agatagaacc tttgctgttc ttgcatacag 300

caatgtacag gaaagggaag cacagtttgg aacaacagca gagatatatg cctatcgaga 360

agaacaggat tttggaattg agatagtgaa agtgaaagca attggaagac aaaggttcaa 420

agtccttgag ctaagaacac agtcagatgg aatccagcaa gctaaagtgc aaattcttcc 480

cgaatgtgtg ttgccttcaa ccatgtctgc agttcaatta gaatccctca ataagtgcca 540

gatatttcct tcaaaacctg tctcaagaga agaccaatgt tcatataaat ggtggcagaa 600

ataccagaag agaaagtttc attgtgcaaa tctaacttca tggcctcgct ggctgtattc 660

cttatatgat gctgagacct taatggacag aatcaagaaa cagctacgtg aatgggatga 720

aaatctaaaa gatgattctc ttccttcaaa tccaatag 758

## Description

The present invention relates to an inhibitor of the cereblon (CRBN) protein for use in the prevention and/or treatment of restless leg syndrome (RLS) and/or diseases associated therewith. The invention also relates to a method of preventing and/or treating restless leg syndrome (RLS) and/or diseases associated therewith comprising administering a pharmaceutically effective amount of an inhibitor of the cereblon (CRBN) protein to a subject in need thereof. Finally, the invention also relates to a method of identifying an inhibitor of the cereblon (CRBN) protein suitable as a lead compound and/or as a medicament for the prevention and/or treatment of restless leg syndrome (RLS) and/or diseases associated therewith.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Restless leg syndrome (RLS) is an enigmatic disease in terms of its phenotypic presentation, genetic basis and underlying pathophysiology. The RLS phenotype is a peculiar composite of sensory and motor symptoms which present with circadian rhythmicity: the symptoms worsen or are only present in the evening or at night and reach their peak intensity between midnight and 2 am in the morning. Due to the chronic nature of the disease, it has long-lasting effects on the patients' mental and physical health. Nightwalkers, as RLS patients call themselves, are forced to move their legs during periods of rest especially in the evening and night to relieve uncomfortable or painful sensations in the deep of the calf. This diurnal variation leads to impaired sleep onset and the periodic leg movements during sleep in the vast majority of patients contribute to severe sleep disruption and a reduced quality of life as a major consequence of the disease (Allen et al., Sleep Med. 4, 101-119 (2003)). There are recognized secondary forms of RLS such as in iron deficiency, pregnancy, and end-stage renal disease and associated morbidity such as increased cardiovascular risk (Winkelmann et al., Sleep Med. 7, 545-552 (2006)). An additional phenomenon not specific to RLS, but observed in 80-90% of patients, are periodic limb movements during sleep (PLMS) (Allen et al., Sleep Med. 15, 860-873 (2014)). RLS is one of the most common neurological disorders with an age-dependent prevalence of up to 10% in the elderly in North America and Europe and affects twice as many females as males (Berger et al., Arch. Int. Med. 164, 196-202 (2004); Allen et al., Arch. Int. Med. 165, 1286-1292 (2005)). 2-3% suffer from severe RLS and require lifelong treatment (Allen et al., Sleep Med. 15, 860-873 (2014)). With the world's population aging, the economic and health burden imposed by RLS will increase, highlighting the need for effective treatment and possibly prevention of the disorder.

Dopaminergic agents originally developed for Parkinson disease provide first line treatment leading to a prompt relief of RLS with an unknown mode of action (Hening et al., Sleep 27, 560-583 (2004)). Neurophysiological, pharmacological and neuroimaging studies suggest that the characteristic symptoms originate in the central nervous system, yet the underlying neurobiology remains obscure (Barriére et al., Prog. Neurobiol. 77, 139-165 (2005); Trenkwalder et al., Lancet Neurol. 4, 465-475 (2005)). Somatosensory perception and motor behavior are controlled on many levels in the central nervous system and the periphery. Disease-causing events can happen on any level and at any time during life. Imbalances in the dopaminergic system, the glutamatergic system and brain iron homeostasis have been described but if and how exactly they interplay in RLS is unknown, as is the importance of both spinal cord and brain structures (Trenkwalder & Paulus, Nat. Rev. Neurol. 6, 337-346 (2010); Allen, R. P., Sleep Med. Clin. 10, 207-14 (2015)). Abnormalities in sensorimotor gating are also discussed as disease mechanism but a clear understanding of the importance of these individual aspects and their possible relationships is still lacking.

The propensity to develop RLS symptoms is strongly influenced by genetic predisposition. Family and twin studies have estimated the heritability to be 50 to 60% (Schormair & Winkelmann, Sleep Med. Clin. 6, 203-215 (2011)). GWAS of moderate sample size (discovery GWAS case sample of 1,000) were the first studies to uncover risk loci for RLS, six in total to date (Winkelmann et al., Nat. Genet. 39, 1000-6 (2007); Stefansson et al., N. Engl. J. Med. 357, 639-47 (2007); Schormair, et al., Nat. Genet. 40, 946-8 (2008); Winkelmann et al., PLoS Genet. 7, e1002171 (2011)). These provided first entry points for functional dissection and finemapping studies, but their limited numbers has hindered a more detailed understanding of the molecular pathways underlying the disease.
Thus, there remains a need to better understand the etiology of RLS as well as to provide alternative treatment and prevention options to RLS patients or subjects at risk of developing RLS.

The technical problem underlying the present invention was to identify alternative and/or improved means and methods to therapeutically address RLS.

The solution to this technical problem is achieved by providing the embodiments characterized in the claims.

Accordingly, the present invention relates in a first embodiment to an inhibitor of the cereblon (CRBN) protein for use in the prevention and/or treatment of restless leg syndrome (RLS) and/or diseases associated therewith.

The term "inhibitor" in accordance with the present invention refers to an inhibitor that reduces or abolishes the biological function or activity of a particular target protein, i.e. the cereblon (CRBN) protein. An inhibitor may perform any one or more of the following effects in order to reduce or abolish the biological function or activity of the protein to be inhibited: (i) the transcription of the gene encoding the protein to be inhibited is lowered, i.e. the level of mRNA is lowered, (ii) the translation of the mRNA encoding the protein to be inhibited is lowered, (iii) the protein performs its biochemical function with lowered efficiency in the presence of the inhibitor, and (iv) the protein performs its cellular function with lowered efficiency in the presence of the inhibitor.

Compounds falling in class (i) include compounds interfering with the transcriptional machinery and/or its interaction with the promoter of said gene and/or with expression control elements remote from the promoter such as enhancers. Compounds of class (ii) comprise antisense constructs and constructs for performing RNA interference (e.g. siRNA) well known in the art (see, e.g. Zamore (2001) Nat Struct Biol. 8(9), 746; Tuschl (2001) Chembiochem. 2(4), 239). Compounds of class (iii) interfere with the molecular function of the protein to be inhibited, in the case of cereblon its biological activites and functions, in particular at least one of the preferred activities and functions (a) to (d) described herein below. Accordingly, active site binding compounds are envisaged. Class (iv) includes compounds which do not necessarily bind directly to the target, but still interfere with its function or activity, for example by binding to and/or inhibiting the function of or inhibiting expression of members of a pathway which comprises the target. These members may be either upstream or downstream of the target within said pathway. For example, such compounds may alter the affinity or rate of binding of a known ligand to the receptor or compete with a ligand for binding to the receptor or displace a ligand bound to the receptor.

Preferably, the inhibitor is an inhibitor of classes (iii) and (iv). More preferred, the inhibitor is an active-site binder, i.e. an inhibitor of class (iii), and as such binds to an active site on the cereblon protein. The term "active site" in relation to proteins is well-known in the art and includes binding sites and catalytic sites. Active sites on the cereblon protein are known in the art and some are described herein below as preferred, namely the active site for (a) binding to MEIS2, (b) binding to the damaged DNA binding protein 1 (DBB1), (c) binding to BK_{Ca} channels, and/or (d) binding to the voltage-gated chloride channel CIC-2.

The inhibitor, in accordance with the present invention, may in certain embodiments be provided as a proteinaceous compound or as a nucleic acid molecule encoding the inhibitor. For example, the nucleic acid molecule encoding the inhibitor may be incorporated into an expression vector comprising regulatory elements, such as for example specific promoters, and thus can be delivered into a cell. Methods for targeted transfection of cells and suitable vectors are known in the art, see for example Sambrook and Russel ("Molecular Cloning, A Laboratory Manual", Cold Spring Harbor Laboratory, N.Y. (2001)). Incorporation of the nucleic acid molecule encoding the inhibitor into an expression vector allows to either selectively or permanently elevate the level of the encoded inhibitor in any cell or a subset of selected cells of a recipient. Thus, a tissue- and/or time-dependent expression of the inhibitor can be achieved, for example restricted to cells of the brain and/or spinal cord.

The cereblon (CRBN) protein to be inhibited by the inhibitor is the human CRBN protein which is known in the art and described, for example, in Hyoung Kyu Kim, Tae Hee Ko, Bayalagmaa Nyamaa et al, Pflugers Arch Eur J Physiolo 2016 468:1299-1309 and Xiu-Bao Chang A, Keith Stewart. Int J Biochen Mol Biol 2011;2(3):287-294. It is encoded by the CRBN gene located on the human chromosome 3, on the short arm at position p26.3 and has the sequence as depicted in SEQ ID NOs: 1 and 2, encoded by the mRNAs of SEQ ID NOs: 4 and 5 for isoform 1 and isoform 2 of cereblon. There are also different transcripts known to be transcribed from the cereblon gene (such as, e.g., depicted in SEQ ID NO: 3 (protein) and 4 (mRNA)). In accordance with the invention, the cereblon protein referred to herein includes all known variants and also those that may potentially be identified in the future, e.g. mutated cereblon proteins due to additions, deletions and/or substitutions of amino acids. It is understood that the known cereblon proteins can be targeted by the inhibitor as defined herein and display the biological activities and functions of cereblon described herein, preferably at least one of the preferred biological activities and functions (a) to (d) mentioned below, most preferred the activity and function of cereblon to bind to MEIS2. In other terms, the cereblon protein can function as pharmaceutical target for the prevention and/or treatment of RLS and/or diseases associated therewith. Preferably, the cereblon protein is cereblon expressed/located in the brain. Also preferred is that the cereblon protein is that of SEQ ID NOs: 1 and 2, encoded by the mRNA sequence of SEQ ID NOs: 4 and 5. Generally, the human protein and mRNA sequences can be retrieved from the database maintained online by the National Center for Biotechnology Information (NCBI), 8600 Rockville Pike, Bethesda MD, 20894 USA, using, e.g., the accession number NP_057386 (version NP_057386.2; GI:39545580) or NP_001166953 (version NP_001166953.1; GI: 291045198) (protein sequences, released 15-MAR-2015)). The cereblon protein has been shown to be a cofactor of damaged DNA-binding protein (DBB1) that acts as the central component of an E3 ubiquitin ligase complex and, thus, regulates the selective degradation of key proteins in DNA repair, replication and transcription (lovine et al., Int J Biochem Cell Biol 43: 1664-1667 (2011)). It has also been shown that CRBN is involved in energy metabolism and negatively regulates AMP-activated protein kinase signaling. Furthermore, CRBN was found to bind to large-conductance calcium-activated potassium (BK_{Ca}) channels (Jo et al., J Neurochem 94:1212-1224 (2005)). In addition, CRBN also binds to the voltage gated chloride channel-2 (CLC-2) which is involved in regulating resting membrane potential, cell volume, intracelluar PH and cell proliferation and differentiation. Through unbiased screens the homeobox transcription factor MEIS2 has been identified as an endogenous substrate to the complex DDB1-CRBN.

The term "inhibitor of the cereblon (CRBN) protein" in accordance with the present invention refers to an inhibitor that reduces the biological function or activity of the cereblon protein. Biological function or activity denotes in particular any known biological function or activity of cereblon and also including those elucidated in accordance with the present invention. Preferably, said biological function or activity is cereblon's capability of (a) binding to MEIS2, (b) its capability of binding the damaged DNA binding protein 1 (DBB1), (c) its capability of binding to BK_{Ca} channels, and/or (d) its capability of binding to the voltage-gated chloride channel CIC-2. All these functions or activities can be tested for either using any of a variety of standard methods known in the art, such as yeast two-hybrid screening, affinity purification coupled to mass spectrometry, protein microarrays, resonance energy transfer systems, or further methods described herein, and/or with the teachings of the documents cited therein.

In a preferred embodiment, the inhibitor reduces at least one biological function or activity of cereblon, preferably at least one of the preferred activities or functions, namely cereblon's capability of binding to MEIS2, its capability of binding the damaged DNA binding protein 1 (DBB1), its capability of binding to BK_{Ca} channels, and/or its capability of binding to the voltage-gated chloride channel CIC-2, by at least 50%, preferably by at least 75%, more preferred by at least 90% and even more preferred by at least 95% such as at least 98% or even by 100% as compared to the biological function or activity in the absence of said inhibitor. The term reduction by at least, for example 75%, refers to a decreased biological function or activity such that cereblon looses 75% of at least one function or activity recited herein, in particular the preferred activities and functions, and, consequently, has only 25% of the function or activity remaining as compared to a cereblon protein that is not inhibited. Also preferred, biological function or activity of cereblon drops to less than 10⁻², less than 10⁻³, less than 10⁻⁴ or less than 10⁻⁵ times the biological function or activity compared to the biological function or activity in the absence of said inhibitor. As outlined herein above, the reduction of the biological function or activity of cereblon is mediated by inhibitors using different mechanisms of actions. Depending on said mechanism of action, a reduction of, e.g., 75% may be achievable by a given inhibitor by reducing the biological function or activity of all or substantially all cereblon proteins by 75% or by fully inhibiting 75% of all or substantially all cereblon proteins. In other words, the reduction of said biological function or activity may be of qualitative or quantitative nature. The term "substantially all" is meant to specify that at least 95% or more of the cereblon proteins are encompassed. The use of the term "substantially all" is a tribute to the constant changes of protein expression seen in a cell.

The function of any of the inhibitors referred to in the present invention may be identified and/or verified by using, e.g., high throughput screening assays (HTS). High-throughput assays, independently of being biochemical, cellular or other assays, generally may be performed in wells of microtiter plates, wherein each plate may contain, for example 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to the observed biological activity.

The determination of the binding of potential inhibitors can be effected in, for example and without limitation, any binding assay, preferably biophysical binding assay, which may be used to identify binding of test molecules prior to performing the functional/activity assay with the inhibitor. Suitable biophysical binding assays are known in the art and comprise fluorescence polarization (FP) assay, fluorescence resonance energy transfer (FRET) assay and surface plasmon resonance (SPR) assay. Instead of or in addition to assessing the direct interaction of inhibitor and target molecule by binding assays, one may indirectly determine the interaction of the inhibitor with its target molecule by using a suitable read out. For example, in cases where the inhibitor acts by decreasing the expression level of the target protein, the determination of the expression level of the protein can, for example, be carried out on the nucleic acid level or on the amino acid level.

Methods for determining the expression of a protein on the nucleic acid level include, but are not limited to, northern blotting, PCR, RT-PCR or real RT-PCR.
A northern blot allows the determination of RNA or isolated mRNA in a sample. Northern blotting involves the use of electrophoresis to separate RNA samples by size and detection with a hybridization probe complementary to part of or the entire target sequence. Initially, total RNA extraction from the sample is performed. If desired, mRNA can be separated from said initial RNA sample through the use of oligo (dT) cellulose chromatography to isolate only the RNA with a poly(A) tail. RNA samples are then separated by gel electrophoresis. The separated RNA is then transferred to a nylon membrane through a capillary or vacuum blotting system. After transfer to the membrane, the RNA is immobilized through covalent linkage to the membrane by, e.g., UV light or heat. Then, the RNA is detected using suitably labeled probes and X-ray film and can subsequently be quantified by densitometry. Suitable compositions of gels, buffers and labels are well-known in the art and may vary depending on the specific sample and target to be identified.
PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq polymerase. The primers for the amplification may be labeled or be unlabeled. DNA amplification can be performed, e.g., with a Applied Biosystems Veriti® Thermal Cycler (Life Technologies Corporation, Carlsbad, CA), C1000™ thermal cycler (Bio-Rad Laboratories, Hercules, CA,), or SureCycler 8800 (Agilent Technologies, Santa Clara, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step, e.g. at 55°C for 1 min as well as a final extension step, e.g. at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, it is well known in the art how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and *Thermus thermophilus* DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. *Thermus thermophilus* DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT reaction can be performed, for example, in a 20µl reaction mix containing: 4 µl of 5x AMV-RT buffer, 2 µl of oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case. The resulting products are loaded onto an agarose gel and band intensities are compared after staining the nucleic acid molecules with an intercalating dye such as ethidium bromide or SybrGreen. A lower band intensity of the sample treated with the inhibitor as compared to a non-treated sample indicates that the inhibitor successfully inhibits the protein.
Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor, which was formerly quenched due to the close proximity to the 3' acceptor in the TaqMan probe sequence. Thereby, the process of amplification can be monitored directly and in real time, which permits a significantly more precise determination of expression levels than conventional endpoint PCR. Also of use in real time RT-PCR experiments is a DNA intercalating dye such as SybrGreen for monitoring the de novo synthesis of double stranded DNA molecules.

Methods for the determination of the expression of a protein on the amino acid level include, but are not limited to, western blotting or polyacrylamide gel electrophoresis in conjunction with protein staining techniques such as Coomassie Brilliant blue or silver-staining. The total protein is loaded onto a polyacrylamide gel and electrophoresed. Afterwards, the separated proteins are transferred onto a membrane, e.g. a polyvinyldifluoride (PVDF) membrane, by applying an electrical current. The proteins on the membrane are exposed to an antibody specifically recognizing the protein of interest. After washing, a second antibody specifically recognizing the first antibody and carrying a readout system such as a fluorescent dye is applied. The amount of the protein of interest is determined by comparing the fluorescence intensity of the protein derived from the sample treated with the inhibitor and the protein derived from a non-treated sample. A lower fluorescence intensity of the protein derived from the sample treated with the inhibitor indicates a successful inhibitor of the protein. Also of use in protein quantification is the Agilent Bioanalyzer technique (e.g., Agilent 2100 Bioanalyzer; Agilent Technologies, Santa Clara, CA).

It is understood by the person skilled in the art that the biological activity or function of cereblon which renders it suitable as therapeutic target for the prevention and/or treatment of RLS and/or diseases associated therewith can be inhibited by one or more inhibitor(s), as described herein above, provided that the different inhibitors do not interfere with one another which can be tested in accordance with methods known in the art and/or described herein. It is preferred that the inhibitors provide an additive effect and, optionally, a synergistic effect in their inhibitory activity. Preferably, an inhibitor in accordance with the invention specifically inhibits the biological activity or function of cereblon. The term "specifically" in this context refers to the capability of an inhibitor to not have an effect or an essential effect on other molecules than the target molecule cereblon. In other words, a corresponding inhibitor does not display cross-reactivity or essentially does not display cross-reactivity. In this context, the term "essentially" is meant to refer to an insignificant or negligible effect. The insignificance or negligibility can be based on functional or quantitative parameters. For example, only a minimal amount of cross-reactivity occurs with a different non-target molecule and/or cross-reactivity occurs, however, with a non-target molecule that is present in insignificant amounts and/or the binding of the inhibitor to the non-target molecule is of no consequence.

Preferably, the inhibitor of the present invention is comprised in a pharmaceutical composition, preferably further comprising a pharmaceutically acceptable carrier, excipient and/or diluent.

The term "pharmaceutical composition", as used herein, relates to a composition to be administered to a patient, preferably a human patient. The pharmaceutical composition of the invention comprises at least one, such as at least two, e.g. at least three, in further embodiments at least four such as at last five of the above mentioned inhibitors. In cases where more than one inhibitor is comprised in the pharmaceutical composition it is understood that none of these inhibitors has any or essentially any inhibitory effect on the other inhibitors also comprised in the composition. The term "essentially" in this context refers to an insignificant or negligible inhibitory effect. Again, it is preferred that the inhibitors provide an additive effect and, optionally, a synergistic effect in their inhibitory activity.

The composition may be in solid, liquid or gaseous form and may be, inter alia, in a form of (a) powder(s), (a) tablet(s), (a) solution(s) or (an) aerosol(s).

As mentioned above, it is preferred that said pharmaceutical composition comprises a pharmaceutically acceptable carrier, excipient and/or diluent. Examples of suitable pharmaceutical carriers, excipients and/or diluents are well known in the art and include phosphate buffered saline solutions, water, emulsions, such as oil/water emulsions, various types of wetting agents, sterile solutions etc. The carriers, excipients and diluents to some extent depend on the chemical nature of the actual inhibitors and can be chosen by the skilled person according to established protocols. Compositions comprising such carriers can be formulated by well known conventional methods. These pharmaceutical compositions can be administered to the subject at a suitable dose. Administration of the suitable compositions may be effected by different ways, e.g., by oral, intravenous, intraperitoneal, subcutaneous, intramuscular, topical, intradermal, intranasal or intrabronchial administration. It is particularly preferred that said administration is carried out by injection and/or delivery, e.g., to a site in the bloodstream such as a artery or directly into the respective tissue. The compositions of the invention may also be administered directly to a targeted site, e.g., by biolistic delivery to an external or internal target site. Local administration is preferred over systemic administration to, e.g., minimize the amount of drug used or decrease the risk of adverse side effects, if any. The dosage regimen will be determined by the attending physician and clinical factors. As is well known in the medical arts, dosages for any one patient depend upon many factors, including the patient's size, body surface area, age, the potency and bioavailability of the particular compound to be administered, sex, time and route of administration, general health, and other drugs being administered concurrently. In particular, the potency and mode of action of an inhibitor may dictate not only its dosage, but also its way of administration. For example, inhibitors that due to their mode of action completely abolish the biological activity or function of a cereblon protein when binding to the latter, may not be suitable for systemic administration due to the possible occurrence of unwanted side effects, if parameters such as, e.g., bioavailability cannot be sufficiently controlled in the sense of minimizing given unwanted side effects. Pharmaceutically active matter may be present in amounts between 1 ng and 10 mg/kg body weight per dose; however, doses below or above this exemplary range are envisioned, especially considering the aforementioned factors. If the regimen is a continuous infusion, it should also be in the range of 0.01 µg to 10 mg units per kilogram of body weight per minute. The continuous infusion regimen may be completed with a loading dose in the dose range of 1 ng and 10 mg/kg body weight.

Progress can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. It is particularly preferred that said pharmaceutical composition comprises further agents known in the art to antagonize heart failure. Since the pharmaceutical preparation of the present invention relies on the above mentioned inhibitors, it is preferred that those mentioned further agents are only used as a supplement, i.e. at a reduced dose as compared to the recommended dose when used as the only drug, so as to e.g. reduce side effects conferred by the further agents. Conventional excipients include binding agents, fillers, lubricants and wetting agents.

A "patient" or "subject" for the purposes of the present invention refers to humans. Thus, the methods are applicable to human therapy.

As used herein, the terms "treatment", "treating", "preventing" and "ameliorating" and the like generally mean obtaining a desired pharmacological and/or physiological effect. In the case of prevention, the effect may be prophylactic in terms of completely or partially preventing a disease or symptom thereof. In the case of treatment, the effect may be therapeutic in terms of partially or completely curing a disease and/or adverse effect attributed to the disease. Evidently, there may be cases where, e.g., the treatment of RLS according to the invention will lead to the prevention of another disease that is associated with the treated disease RLS. The term "treatment/treating" as used herein covers any treatment of a disease as referred to herein in a subject and includes: (a) inhibiting RLS and/or disease associated therewith, e.g. arresting its development; or (b) relieving RLS and/or disease associated therewith, e.g. causing regression of RLS and/or disease associated therewith. In accordance with the present invention, the term "prevention" or "preventing" of an disease means the disease *per se* can be hindered of developing or to develop into an even worse situation. Accordingly, it is one aspect of the present invention that the herein described inhibitor of the cereblon protein can be employed in avoidance of RLS and/or disease associated therewith. It is understood that the inhibition of the cereblon protein for the purpose of preventing RLS and/or diseases associated therewith in accordance with the invention will be employed in subjects that are known to have predisposition to develop RLS described herein. For RLS, criteria exist for determining whether a subject has a predisposition to develop RLS. For example, subjects that are predispositioned to develop RLS are identified by assessing, e.g., iron levels (with low iron levels being a risk factor), the presence or absence of genetic variations known to be associated with RLS, e.g., in the BTBD9, MEIS1, MAP2K5/SCOR1, PTPRD and/or TOX3 genes. It is further understood that a preventive administration of an inhibitor of the cereblon protein in accordance with the invention is monitored to avoid occurrence of side effects. Establishing the right dosage and administration regimen for a corresponding preventive treatment can be done without further ado following routine methods known in the art.

The term "RLS" as used in accordance with the present invention comprises all forms of symptoms currently scientifically associated with term "restless legs syndrome". Accordingly, the term "RLS" includes primary and secondary RLS and can be diagnosed as, for example, RLS with intermittent discomfort, RLS with sleep disorder or RLS with perodic limb movement or periodic leg movement. The diagnosis is based on the description of the symptoms by the patient or by a questionnaire including the diagnostic criteria for RLS which have been set up by the International RLS Study group (Becker PM, Sleep Med Clin. 2015 Sep;10(3):235-40; Allen et al., Sleep Med. 2014 Aug;15(8):860-73).

The term "diseases associated therewith" as used in accordance with the present invention relates to diseases which develop in a subject as a consequence of said subject having RLS. In other words, these diseases are or are known to be causally associated with the presence of RLS in a subject. Thus, once RLS is treated or prevented, it can reasonably be expected that also the diseases that the subject developed or might develop due to having RLS can be treated or prevented. Accordingly, the diseases associated with RLS are present in a patient due to being caused by the patient having RLS.

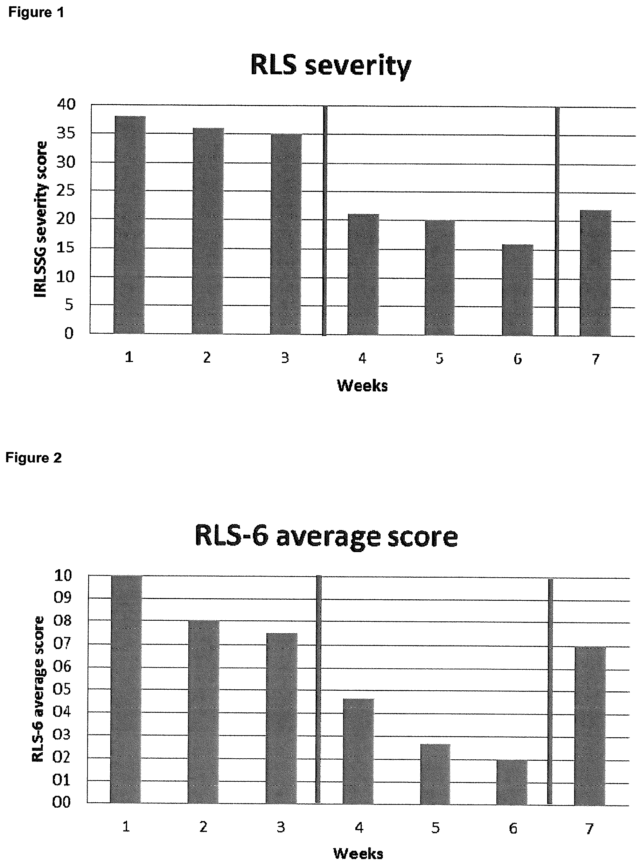

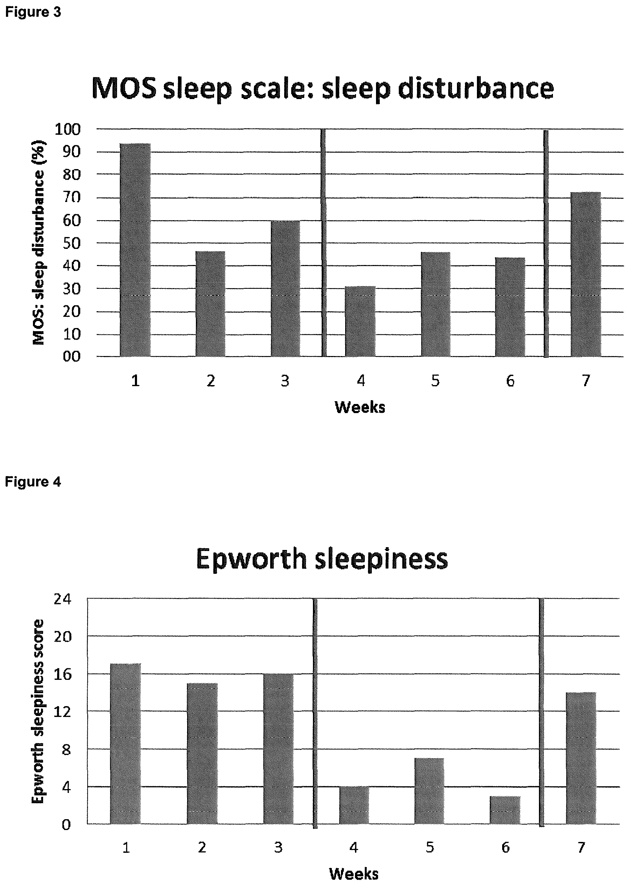

As can be taken from Example 1 herein below, the inventors have conducted a genome-wide association studie (GWAS) for RLS including 6,228 cases and 10,992 controls (see example section for details). While the known risk factors BTBD9, MEIS1, MAP2K5/SCOR1, PTPRD and TOX3 were confirmed as risk factors for RLS, several new risk loci were identified. One of the indentified loci was shown to be located on chromosome 3p26. Based upon research of the genes at said locus, it was found that the cereblon gene is located near said locus on chromosome 3p26. RLS is characterised and classified as a neurodevelopmental disorder. As such, pathways and genes involved in the development of the central nervous system are natural pharmaceutical targets for the treatment of RLS. As described herein above, cereblon is known to be involved in the assembly and expression of BK_{Ca} channels in brain regions involved in memory and learning. Also, cereblon was shown to bind to the voltage-gated chloride channel CIC-2. The function of the central nervous system depends on the correct regulation of ion channels by interacting proteins. Furthermore, cereblon binds to DBB1, thereby forming a Cullin4a RING E3 ubiquitin ligase and acts as the recognition component of said Cullin4a RING E3 ubiquitin ligase. In addition, a natural ligand is MEIS2 which is ubiquitinated via binding to cereblon in its function as Cullin4a RING E3 ubiquitin ligase and results in the degradation of MEIS2. MEIS2 is a paralog of the strongest validated RLS risk marker MEIS1 which in turn is known to be involved in various neurodevelopmental processes including the central nervous system, e.g., via its function as transcriptional regulator of Pax6. In terms of MEIS1, a direct involvement of RLS-associated SNPs in the development of the forebrain has been demonstrated (Spieler et al., Genome Res. 2014 Apr;24(4):592-603). The RLS-associated SNP in MEIS1 is leading to reduced MEIS1 during embryonic development in the ganglionic eminences and reduced MEIS1 levels were shown to be also present in post mortem brain tissue in RLS patients. As with MEIS1, stabilising the amount of MEIS2 can be expected to counteract RLS. Further, cereblon functionally resembles another known RLS risk factor, namely BTBD9. BTBD9 is characterized by the presence of a BTB domain and proteins with such a domain have been identified as the substrate recognizing unit of cullin 3 E3 ubiquitin ligases. In summary, cereblon evidently mediates and is directly involved in several pathways relating to neurodevelopment. Moreover, Example 2 of the present application demonstrates on the basis of the CRBN inhibitor thalidomide that the treatment of an RLS patient with a CRBN inhibitor can significantly reduce RLS symptoms; see results shown in Table 1 and Figures 1 to 4. As such, it can be concluded that cereblon is a pharmaceutical target whose inhibition will result in the prevention and/or treatment of RLS as defined herein.

The present invention further relates to a method of preventing and/or treating restless leg syndrome (RLS) and/or diseases associated therewith comprising administering a pharmaceutically effective amount of an inhibitor of the cereblon (CRBN) protein to a subject in need thereof.

The explanations, definitions and preferred embodiments described herein above for the use of the inhibitor apply *mutatis mutandis* also to the method of treatment of the invention. In particular, it is preferred that the inhibitor is comprised in a pharmaceutical composition as defined above.

In a preferred embodiment of the inhibitor of the invention or the method of the invention, the inhibitor is an antibody or a fragment or derivative thereof, an aptamer, an siRNA, an shRNA, a miRNA, a ribozyme, an antisense nucleic acid molecule, a small molecule or modified versions of these inhibitors.

The term "antibody" as used in accordance with the present invention comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments as well as Fd, F(ab')₂, Fv or scFv fragments; see, for example Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also includes embodiments such as chimeric (human constant domain, non-human variable domain), single chain and humanized (human antibody with the exception of non-human CDRs) antibodies.

Various techniques for the production of antibodies are well known in the art and described, e.g. in Harlow and Lane (1988) and (1999), loc. cit. Thus, the antibodies can be produced by peptidomimetics. Further, techniques described for the production of single chain antibodies (see, inter alia, US Patent 4,946,778) can be adapted to produce single chain antibodies specific for the target of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques are described, e.g. in Harlow and Lane (1988) and (1999), loc. cit. and include the hybridoma technique (as originally demonstrated by Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of cereblon (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

More specifically, aptamers can be classified as nucleic acid aptamers, such as DNA or RNA aptamers, or peptide aptamers. Whereas the former normally consist of (usually short) strands of oligonucleotides, the latter preferably consist of a short variable peptide domain, attached at both ends to a protein scaffold.

Nucleic acid aptamers are nucleic acid species that, as a rule, have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms.

Peptide aptamers usually are peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a -Cys-Gly-Pro-Cys-loop in the wild protein, the two cysteins lateral chains being able to form a disulfide bridge. Peptide aptamer selection can be made using different systems, but the most widely used is currently the yeast two-hybrid system.
Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, fusion to albumin or other half life extending proteins etc. are available to scientists such that the half-life of aptamers can be increased for several days or even weeks.

The term "peptide" as used herein describes a group of molecules consisting of up to 30 amino acids, whereas the term "polypeptide" as used herein describes a group of molecules consisting of more than 30 amino acids. The group of peptides and polypeptides are referred to together with the term "(poly)peptide". Also encompassed by the term "(poly)peptide" are proteins as well as fragments of proteins of more than 30 amino acids. The term "fragment of protein" in accordance with the present invention refers to a portion of a protein comprising at least the amino acid residues necessary to maintain the biological activity of the protein. Preferably, the amino acid chains are linear. (Poly)peptides may further form multimers consisting of at least two identical or different molecules. The corresponding higher order structures of such multimers are correspondingly termed homo- or heterodimers, homo- or heterotrimers etc. Furthermore, peptidomimetics of such (poly)peptides where amino acid(s) and/or peptide bond(s) have been replaced by functional analogues are also encompassed by the invention. Such functional analogues include all known amino acids other than the 20 gene-encoded amino acids, such as selenocysteine. The term "(poly)peptide" also refers to naturally modified (poly)peptides where the modification is effected e.g. by glycosylation, acetylation, phosphorylation and similar modifications which are well known in the art.
It is also well known that (poly)peptides are not always entirely linear. For instance, (poly)peptides may be branched as a result of ubiquitination, and they may be circular, with or without branching, generally as a result of post-translation events, including natural processing event and events brought about by human manipulation which do not occur naturally. Circular, branched and branched circular (poly)peptides may be synthesized by non-translational natural processes and by synthetic methods. The modifications can be a function of how the (poly)peptide is made. For recombinant (poly)peptides, for example, the modifications will be determined by the host cells posttranslational modification capacity and the modification signals in the amino acid sequence. Accordingly, when glycosylation is desired, a (poly)peptide should be expressed in a glycosylating host, generally an eukaryotic cell, for example Cos7, HELA or others. The same type of modification may be present in the same or varying degree at several sites in a given (poly)peptide. Also, a given (poly)peptide may contain more than one type of modification.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene of which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'-overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery *in vivo* through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics). The activity and specificity of siRNAs can be altered by various modifications such as, e.g., by inclusion of a blocking group at the 3' and 5' ends, wherein the term "blocking group refers to substituents of that can be attached to oligonucleotides or nucleomonomers, either as protecting groups or coupling groups for synthesis (cf. WO 98/13526, EP 2221377 B1), by inclusion of agents that enhance the affinity to the target sequence such as intercalating agents (e.g., acridine, chlorambucil, phenazinium, benzophenanthirdine), attaching a conjugating or complexing agent or encapsulating it to facilitate cellular uptake, or attaching targeting moieties for targeted delivery.

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules which, as endogenous RNA molecules, regulate gene expression. Binding to a complementary mRNA transcript triggers the degradation of said mRNA transcript through a process similar to RNA interference. Accordingly, miRNA may be employed as an inhibitor of cereblon.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Non-limiting examples of well-characterized small self-cleaving RNAs are the hammerhead, hairpin, hepatitis delta virus, and *in* vitro-selected lead-dependent ribozymes, whereas the group I intron is an example for larger ribozymes. The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity *in vitro* and in some cases also *in vivo.* The best results are usually obtained with short ribozymes and target sequences.

A recent development, also useful in accordance with the present invention, is the combination of an aptamer recognizing a small compound with a hammerhead ribozyme. The conformational change induced in the aptamer upon binding the target molecule is supposed to regulate the catalytic function of the ribozyme.

The term "antisense nucleic acid molecule" is known in the art and refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

A "small molecule" as used herein may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as less than about 500 amu, and even more preferably less than about 250 amu. The size of a small molecule can be determined by methods well-known in the art, e.g., mass spectrometry. The small molecules may be designed, for example, based on the crystal structure of the target molecule, where sites presumably responsible for the biological activity, can be identified and verified in *in vivo* assays such as *in vivo* high-throughput screening (HTS) assays.

The term "modified versions of these inhibitors" in accordance with the present invention refers to versions of the inhibitors that are modified to achieve i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised application form and route by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (k) conversion of alkyl substituents to cyclic analogues, or (l) derivatisation of hydroxyl groups to ketales, acetales, or (m) N-acetylation to amides, phenylcarbamates, or (n) synthesis of Mannich bases, imines, or (o) transformation of ketones or aldehydes to Schiff's bases, oximes, acetales, ketales, enolesters, oxazolidines, thiazolidines; or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

In a further preferred embodiment of the inhibitor of the invention or the method of the invention, the inhibitor inhibits: (a) the binding of the cereblon (CRBN) protein to the ligand MEIS2; (b) the binding of the cereblon (CRBN) protein to the damaged DNA binding protein 1 (DBB1); (c) the binding of the cereblon (CRBN) protein to BK_{Ca} channels; and/or (d) the binding of the cereblon (CRBN) protein to the voltage-gated chloride channel CIC-2.

It is preferred that the inhibitor inhibits specific biological activities and functions as identified in alternatives (a) to (d). It is in this regard also preferred that in this embodiment the inhibitor is an active site inhibitor, namely an inhibitor that directly interacts with the cereblon protein to achieve the inhibition of cereblon to bind to MEIS2, DBB1, BK_{Ca} channels and/or the voltage-gated chloride channel CIC-2. It is preferred that a corresponding inhibitor is an inhibitor that is an antibody or a fragment or derivative thereof, an aptamer, ribozyme, a small molecule or modified versions of these inhibitors.

Inhibition of cereblon binding MEIS2 can, e.g., be achieved by blocking its binding site on cereblon. The binding site of MEIS2 is considered to be the same as the binding site identified for the small molecule thalidomide, which molecule is discussed herein below in detail. The location and structure of this binding site is known in the art and has been described, e.g., by Lupas et al. (2015) (PLoS Comput Biol 11(1): e1004023. doi:10.1371/journal.pcbi.1004023; see also, e.g., Fischer et al. (2015), Nature 512(7512):49-53), as positioned at the C-terminus. Thus, making this binding site unavailable for MEIS2 by use of an inhibitor will result in the inhibition of the binding of cereblon to MEIS2. Further, it is also envisaged to block the interaction site on MEIS2 with which it binds to cereblon.

Also the inhibition of cereblon binding DBB1 can, e.g., be achieved by blocking its binding site on cereblon. The binding site of DBB1 is also well-known in the art (see, e.g., Fischer et al. (2015), Nature 512(7512):49-53) and can thus be targeted without further ado by an inhibitor. Further, it is also envisaged to block the interaction site on DBB1 with which it binds to cereblon.

The term "BK_{Ca} channels" (also known as High-conductance voltage- and Ca²⁺-activated K⁺ (BK) channels or Slo1 channels) as used herein relates to the well-known voltage-dependent potassium-selective ion channels whose activity is regulated by intracellular calcium (see, e.g., Miller C (2000), Genome Biol 1(4): reviews0004.1-reviews0004.5; Yuan et al., Science Jul 9, 329(5988):182-186 (2010)). Cereblon was found to bind to the α subunit of BK_{Ca} channels and suppress their membrane expression, suggesting that cereblon modulates BK_{Ca} channel assembly and translocation (see, e.g., Higgins et al., Neurogenetics 9:219-223 (2008); Higgins et al., J Neurogenet 24:18-26 (2010); Jo et al., J Neurochem 94:1212-1224 (2005); Kim et al., Pflugers Arch - Eur J Physiol (2016) 468:1299-1309)). In the brain, these channels play an important role in learning and memory, and BK_{Ca} channel dysfunction is also associated with autism and mental retardation. Since the binding site is known, it can thus be targeted without further ado by an inhibitor. Further, it is also envisaged to block the interaction site on BK_{Ca} channels with which they bind to cereblon.

The voltage-gated chloride channel CIC-2 is known in the art as is its binding to cereblon, namely a distal region of the CIC-2 C-terminus interacts with the Lon domain of cereblon (Hohbereger and Enz, FEBS Lett. 2009 Feb 18;583(4):633-7; Jentsch et al., J Physiol. 1995 Jan;482:19S-25S). Since the binding site is known, it can thus be targeted without further ado by an inhibitor. Further, it is also envisaged to block the interaction site on the CIC-2 channel with which it binds to cereblon.

In a more preferred embodiment of the inhibitor of the invention or the method of the invention, the inhibitor inhibiting the binding of the cereblon (CRBN) protein to the ligand MEIS2 is selected from 2-(2,6-dioxopiperidin-3-yl)-2,3-dihydro-1H-isoindole-1,3-dione, (RS)-3-(4-Amino-1-oxo-1,3-dihydro-2H-isoindol-2-yl)piperidine-2,6-dione, (RS)-4-Amino-2-(2,6-dioxopiperidin-3-yl)isoindole-1,3-dione.

In accordance with the invention, the above listed small molecule inhibitors are active site inhibitors directly interacting with the cereblon protein to inhibit binding of MEIS2 to cereblon.

2-(2,6-dioxopiperidin-3-yl)-2,3-dihydro-1H-isoindole-1,3-dione (IUPAC name) is well-known in the art as "thalidomide" (or also α-(N-Phthalimido)glutarimide) and has been used in medicine for the treatment of various diseases since more than 50 years. Thalidomide has been shown to bind to cereblon in a competitive manner with MEIS2, the natural ligand of cereblon, and prevent substrate degradation while simultaneously activating the DDB1-cereblon complex to recruit transcription factors IKZF1 and IKZF3 to promote their degradation (see, e.g., Ito et al., Science 2010, 327:1345-50; Fischer et al., Nature 2014, 512:49-53; Chamberlain et al., Nat Struct Mol Biol 2014, 21:803-9). In Example 2 of the present application thalidomide is used to treat a patient with RLS. The symptoms of RLS were significantly reduced by the thalidomide treatment; see results shown in Table 1 and Figures 1 to 4. Example 2 proves that an inhibitor of cereblon (CRBN) protein can be used to treat or prevent RLS.

(RS)-3-(4-Amino-1-oxo-1,3-dihydro-2H-isoindol-2-yl)piperidine-2,6-dione (IUPAC name) is also well-known in the art being a thalidomide analogue and is commonly known as "lenalidomide" and was introduced in 2004, mainly for the treatment of multiple myeloma as an alternative for thalidomide.

(RS)-4-Amino-2-(2,6-dioxopiperidin-3-yl)isoindole-1,3-dione (IUPAC name) is equally well-known in the art being a thalidomide analogue and is commonly known as "pomalidomide". As its parent compound it has anti-angiogenic properties and also acts as immunomodulator.

Both, lenalidomide and pomalidomide - as thalidomide - have been shown to bind to cereblon in a competitive manner with MEIS2, i.e. MEIS2 cannot bind to cereblon when lenalidomide or pomalidomide (or thalidomide) are bound to cereblon.

The above mentioned small molecules include their salts and solvates, but can also relate to the compounds *per se.*

Most preferred is that the inhibitor inhibiting the binding of the cereblon (CRBN) protein to the ligand MEIS2 is 2-(2,6-dioxopiperidin-3-yl)-2,3-dihydro-1H-isoindole-1,3-dione, i.e. thalidomide. Thus, most preferred is that the invention relates to 2-(2,6-dioxopiperidin-3-yl)-2,3-dihydro-1H-isoindole-1,3-dione, i.e., thalidomide, for use in the prevention and/or treatment of restless leg syndrome (RLS) and/or diseases associated therewith. Evidently, the explanations, definitions and preferred embodiments described herein above for the use of the inhibitor in general apply *mutatis mutandis* also to this most preferred embodiment.

Accordingly, it is also most preferred that the invention relates to a method of preventing and/or treating restless leg syndrome (RLS) and/or diseases associated therewith comprising administering a pharmaceutically effective amount of 2-(2,6-dioxopiperidin-3-yl)-2,3-dihydro-1H-isoindole-1,3-dione to a subject in need thereof.

While mentioned herein as preferred inhibitors inhibiting the binding of the cereblon (CRBN) protein to the ligand MEIS2, it is alternatively also envisaged that (RS)-3-(4-Amino-1-oxo-1,3-dihydro-2H-isoindol-2-yl)piperidine-2,6-dione and (RS)-4-Amino-2-(2,6-dioxopiperidin-3-yl)isoindole-1,3-dione are not inhibitors of the cereblon protein to be used in the prevention and/or treatment of restless leg syndrome (RLS) and/or diseases associated therewith. In other terms, the invention also relates to an inhibitor of the cereblon (CRBN) protein for use in the prevention and/or treatment of restless leg syndrome (RLS) and/or diseases associated therewith, wherein the inhibitor, including the inhibitor that inhibits the binding of the cereblon (CRBN) protein to the ligand MEIS2, is not (RS)-3-(4-Amino-1-oxo-1,3-dihydro-2H-isoindol-2-yl)piperidine-2,6-dione and (RS)-4-Amino-2-(2,6-dioxopiperidin-3-yl)isoindole-1,3-dione.

In another preferred embodiment, the diseases associated with RLS are depression, neuroticism, and/or periodic limb movement disorder.

Depression associated with RLS, i.e. resulting from the presence of RLS in the patient, is characterised by any depressive symptoms including, for example, depressed mood, sleep disturbances, awakening during the nights, weight gain and/or listlessness.

Neuroticism associated with RLS, i.e. resulting from the presence of RLS in the patient, is characterised by behaviors including, for example, anxiety, fear, moodiness, worry, envy, and/or frustration.

Periodic limb movement disorder associated with RLS, i.e. resulting from the presence of RLS in the patient, is characterised by the occurrence of periodic limb movements during sleep and wakefulness. These can, for example, be diagnosed by polysomnographic studies or portable devices.

In another embodiment, the invention relates to a method of identifying an inhibitor of the cereblon (CRBN) protein suitable as a lead compound and/or as a medicament for the prevention and/or treatment of restless leg syndrome (RLS) and/or diseases associated therewith, comprising the steps of: (a) determining the level of cereblon (CRBN) protein, the level of cereblon (CRBN) transcript and/or the level of activity of cereblon (CRBN) in a cell; (b) contacting said cell or a cell of the same cell population with a test compound; (c) determining the level of cereblon (CRBN) protein, the level of cereblon (CRBN) transcript and/or the level of activity of cereblon (CRBN) in said cell after contacting with the test compound; and (d) comparing the level of cereblon (CRBN) protein, the level of cereblon (CRBN) transcript and/or the level of activity of cereblon (CRBN) determined in step (c) with the cereblon (CRBN) protein, the cereblon (CRBN) transcript and/or the cereblon (CRBN) activity level determined in step (a), wherein a decrease of cereblon (CRBN) protein, the cereblon (CRBN) transcript and/or the cereblon (CRBN) activity level in step (c) as compared to step (a) indicates that the test compound is an inhibitor of the cereblon (CRBN) protein suitable as a lead compound and/or as a medicament for the prevention and/or treatment of RLS and/or diseases associated therewith.

This embodiment relates to a cellular screen, wherein inhibitors may be identified which exert their inhibitory activity by interfering with the expression of cereblon, either by affecting the stability (half-life) of the cereblon protein or cereblon transcript (mRNA) or by interfering with the transcription or translation of cereblon.

The inhibitor can be any of the inhibitors defined above, i.e. an antibody or a fragment or derivative thereof, an aptamer, a siRNA, a shRNA, a miRNA, a ribozyme, an antisense nucleic acid molecule, modified versions of these inhibitors or a small molecule. The inhibitor may further be, for example a (poly)peptide such as a soluble peptide, including Ig-tailed fusion peptides and members of random peptide libraries (see, e.g., Lam et al. (1991) Nature 354: 82-84; Houghten et al. (1991) Nature 354: 84-86) and combinatorial chemistry-derived molecular libraries made of D-and/or L-configuration amino acids or phosphopeptides (e.g., members of random and partially degenerate, directed phosphopeptide libraries, see, e.g., Songyang et al. (1993) Cell 72: 767-778).

The term "said cell or a cell of the same cell population" as used herein refers either to the cell used in step (a) (first alternative of the term , i.e. "said cell") or to a cell being of the same origin as the cell of step (a) and that is identical or essentially identical in its characteristics to the cell of (a) (second alternative of the term, i.e. "a cell of the same cell population"). Furthermore, this term also encompasses cell populations, such as for example homogenous or essentially homogenous cell populations consisting of cells having identical or essentially identical characteristics, and, thus, is not restricted to single cell analyses. "Essentially identical" means certain differences may exist which are, however, negligible or insignificant with regard to the performance of the cell in the method of the invention. The term "essentially homogenous" is meant to refer to those instances where it is not possible to generate an entirely pure (homogenous) cell population from a tissue or cell colony due to technical restrictions. In corresponding cell populations the vast majority of cells is homologous, while only a negligible or insignificant number of cells are different from said vast majority. The same limitations and definitions with regard to the term "cell" given herein above apply also to this embodiment *mutatis mutandis.*

In accordance with this method of the invention is that step (a) refers to the actual execution of a cell based assay to determine the level of cereblon protein, the level of cereblon transcript and/or the level of activity of cereblon in a cell, i.e. performing a cellular assay. However, step (a) is to also relate to the scenario in which step (a) means that the levels are determined in the form of using established standard values for these levels that have been determined in a cell. "Established standard values" are values that have previously been generated in the respective cells used in the assay in the absence of a test compound, i.e. in untreated cells, and are at the disposal of the person implementing the method of the invention. A corresponding setup may prove beneficial in particular in view of high throughput screenings (HTS).

The cell in this embodiment can be any animal cell. It is understood by the person skilled in the art that depending on the goal to be achieved with the method described, different cells may be more suitable than others. Preferably, the cell is a mammalian cell, more preferred a human cell which is not a human embryonic stem cell. The term "mammalian cell" as used herein, is well known in the art and refers to any cell belonging to an animal that is grouped into the class of mammalia. The term "cell" as used herein can refer to a single and/or isolated cell or to a cell that is part of a multicellular entity such as a tissue, an organism or a cell culture. In other words the method can be performed *in vivo, ex vivo* or *in vitro.* Depending on the particular goal to be achieved with the method of the invention, cells of different mammalian subclasses such as prototheria or theria may be used. For example, within the subclass of theria, preferably cells of animals of the infraclass eutheria, more preferably of the order primates, artiodactyla, perissodactyla, rodentia and lagomorpha are used in the method of the invention. Furthermore, within a species one may choose a cell to be used in the method of the invention based on the tissue type and/or capacity to differentiate equally depending on the goal to be achieved by altering the genome. Three basic categories of cells make up the mammalian body: germ cells, somatic cells and stem cells. A germ cell is a cell that gives rise to gametes and thus is continuous through the generations. Stem cells can divide and differentiate into diverse specialized cell types as well as self renew to produce more stem cells. In mammals there are two main types of stem cells: embryonic stem cells and adult stem cells. Somatic cells include all cells that are not a gametes, gametocytes or undifferentiated stem cells. The cells of a mammal can also be grouped by their ability to differentiate. A totipotent (also known as omnipotent) cell is a cell that is able to differentiate into all cell types of an adult organism including placental tissue such as a zygote (fertilized oocyte) and subsequent blastomeres, whereas pluripotent cells, such as embryonic stem cells, cannot contribute to extraembryonic tissue such as the placenta, but have the potential to differentiate into any of the three germ layers endoderm, mesoderm and ectoderm. Multipotent progenitor cells have the potential to give rise to cells from multiple, but limited number of cell lineages. Further, there are oligopotent cells that can develop into only a few cell types and unipotent cells (also sometimes termed a precursor cell) that can develop into only one cell type. There are four basic types of tissues: muscle tissue, nervous tissue, connective tissue and epithelial tissue that a cell to be used in the method of the invention can be derived from, such as for example hematopoietic stem cells or neuronal stem cells. To the extent human cells are envisaged for use in the method of the invention, it is preferred that such human cell is not obtained from a human embryo, in particular not via methods entailing destruction of a human embryo. On the other hand, human embryonic stem cells are at the skilled person's disposal such as taken from existent embryonic stem cell lines commercially available. Accordingly, the present invention may be worked with human embryonic stem cells without any need to use or destroy a human embryo. Alternatively, or instead of human embryonic stem cells, pluripotent cells that resemble embryonic stem cells such induced pluripotent stem (iPS) cells may be used, the generation of which is state of the art (Hargus G et al., Proc Natl Acad Sci U S A 107:15921-15926; Jaenisch R. and Young R., 2008, Cell 132:567-582; Saha K, and Jaenisch R., 2009, Cell Stem Cell 5:584-595.

Cells to be used may originate from established cell lines but may also include cells of a primary cell line established from a tissue sample. Preferably, the cells originate from the same cell line or are established from the same tissue. Methods to obtain samples from various tissues and methods to establish primary cell lines are well-known in the art (Jones GE, Wise CJ., "Establishment, maintenance, and cloning of human dermal fibroblasts." Methods Mol Biol. 1997;75:13-21). Suitable cell lines may also be purchased from a number of suppliers such as, for example, the American tissue culture collection (ATCC), the German Collection of Microorganisms and Cell Cultures (DSMZ) or PromoCell GmbH, Sickingenstr. 63/65, D-69126 Heidelberg.

As outlined above, certain cells may be more suitable than others to determine cereblon expression levels. For example, the phenotype and physiological state of a specific cell may be more suitable to achieve a pronounced decrease in cereblon expression. For example, some cells may endogenously not display a very high cereblon expression level so that the effects of test compounds cannot be decisively determined. Therefore, preferably cells with a cereblon expression level that is well above the detection threshold of the method for determining expression level so that an accurate determination of the effect of a given test compound can be determined. Alternatively, the cereblon expression level may be artificially increased in cells endogenously expressing cereblon at a low level prior to using corresponding cells in the method of the invention. Also, cells may be genetically engineered to express cereblon or express cereblon in sufficient amounts.

Preferred cell types that may be used in accordance with the invention are central nervous system cells, or cells related to or originating from the central nervous system.

As described hereinabove, cereblon is a therapeutic target for the prevention and/or treatment of RLS and/or disease associated therewith. Therefore, the use of cereblon as a target for the discovery of inhibitors suitable for the treatment and/or prevention RLS and/or disease associated therewith as defined herein is also encompassed by the present invention. It is envisaged that, for example, a decrease of expression levels of cereblon conferred by an inhibitor as described above contributes to the prevention and/or treatment of RLS as defined herein. Accordingly, measurement of the cereblon protein or cereblon transcript level may be used as a readout of the above-described assay.

For example, the above-mentioned cell may exhibit a detectable level of cereblon protein or cereblon transcript before contacting with the test compound and the level of cereblon protein or cereblon transcript may be lower or undetectable after contacting the cell with the test compound, indicating an inhibitor suitable as a lead compound and/or as a medicament for the prevention and/or treatment of restless leg syndrome (RLS) as defined herein and/or diseases associated therewith as defined herein. Preferably, the level of cereblon protein or cereblon transcript after contacting the cell with the test compound is reduced by, for example, at least 10, at least 20, at least 30, at least 40 or at least 50% as compared to the level of cereblon protein or cereblon transcript before contacting the cell with the test compound. More preferably, the level of cereblon protein or cereblon transcript after contacting the cell with the test compound is reduced by, for example, at least 60, at least 70, at least 80, at least 90 or at least 95% as compared to the level of cereblon protein or cereblon transcript before contacting the cell with the test compound. Most preferably, the level of cereblon protein or cereblon transcript after contacting the cell with the test compound is reduced by 100% as compared to the level of cereblon or cereblon before contacting the cell with the test compound. The term "the level of cereblon protein or cereblon transcript is reduced by (at least)...%" refers to a relative decrease compared to the level of cereblon protein or cereblon transcript before contacting the cell with the test compound. For example, a reduction of at least 40% means that after contacting the cell with the test compound the remaining level of cereblon protein or cereblon transcript is only 60% or less as compared to the level of cereblon protein or cereblon transcript before contacting the cell with the test compound. A reduction by 100% means that no detectable level of cereblon protein or cereblon transcript remains after contacting the cell with the test compound. The same applies *mutatis mutandis* to the level of activity of cereblon in the cell.

Measurements of cereblon activity, cereblon protein levels as well as of cereblon transcript level can be accomplished in several ways, as described above.

In a preferred embodiment, the method is carried out *in vitro. In vitro* methods offer the possibility of establishing high-throughput assays, as described above.

In a preferred embodiment, the method of the invention further comprises a step (e) of validating the capability of the inhibitor identified in step (d) to serve as a lead compound and/or as a medicament for the prevention and/or treatment of restless leg syndrome (RLS).

In this preferred embodiment, the inhibitor of the cereblon (CRBN) protein suitable as a lead compound and/or as a medicament for the prevention and/or treatment of restless leg syndrome (RLS) and/or diseases associated therewith identified by the method of the invention is further validated as regards its suitability of being a lead compound and/or as a medicament for the prevention and/or treatment of restless leg syndrome (RLS) and/or diseases associated therewith. Validation as used herein refers to the determination whether the inhibitor identified by the method of the invention displays therapeutic efficacy to warrant its use as lead compound and/or medicament. As such, the validation step can include determining the therapeutic efficacy of the inhibitor identified by the method of the invention in RLS patients having been administered said inhibitor. Alternatively, the validation step can include determining the therapeutic efficacy of the inhibitor identified by the method of the invention outside of RLS patients, e.g., in an animal model for RLS. A corresponding animal model are various types of MEIS1 knock-out animal models or other transgenic animal models with reduced/altered MEIS1 expression/function, either in the CNS or the entire animal. Preferably, the animal model is a murine animal model. Therapeutic effectiveness can be evaluated by different screenings testing the behavior in either altered sleep/wake behavior, locomotion, circadian rhythm or prepulse inhibition and startel reflex.

In a further preferred embodiment, the method of the invention further comprising optimising the pharmacological properties of an inhibitor of the cereblon (CRBN) protein identified as lead compound and/or as a medicament for the prevention and/or treatment of restless leg syndrome (RLS).

The identified so-called lead compounds may be optimized to arrive at a compound which may be used in a pharmaceutical composition. Methods and tools for the optimization of the pharmacological properties of compounds identified in screens, the lead compounds, are known in the art. For example, *in silico* tools for optimizing lead compounds are known in the art and described, e.g., in Cruciani et al., European Journal of Pharmaceutical Sciences, vol. 11, suppl. 2, p. S29-S39 (2000). Furthermore, high-throughput approaches for evaluating properties of lead compounds have been described in Tarbit and Berman, Current Opinion in Chemical Biology, vol. 2, issue 3, p. 411-416 (1998).

In a more preferred embodiment of the method of the invention, the optimisation comprises modifying the inhibitor of cereblon (CRBN) protein to achieve: i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised application form and route, by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (l) conversion of alkyl substituents to cyclic analogues, or (m) derivatisation of hydroxyl groups to ketales, acetales, or (n) N-acetylation to amides, phenylcarbamates, or (o) synthesis of Mannich bases, imines, or (p) transformation of ketones or aldehydes to Schiffs bases, oximes, acetales, ketales, enolesters, oxazolidines, thiazolidines, (q) or combinations thereof.

The various steps recited above are generally known in the art, as described above.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply mutatis mutandis to all attached claims.

The figures show.
**Figure 1** IRLS severity score of the patient at baseline (weeks 1-3), during thalidomide treatment (weeks 4-6) and immediately after finishing treatment (week 7)
**Figure 2** RLS-6 severity of the patient at baseline (weeks 1-3), during thalidomide treatment (weeks 4 - 6) and immediately after finishing treatment (week 7)
**Figure 3** Sleep disturbance scope of patient at baseline (weeks 1-3), during thalidomide treatment (weeks 4-6) and immediately after finishing treatment (week 7)
**Figure 4** Daytime sleepiness of the patient on Epworth Sleepiness Scale at baseline (weeks 1-3), during thalidomide treatment (weeks 4 - 6) and immediately after finishing treatment (week 7)

The example illustrates the invention:

### Example 1:

### 1.1 Genome-wide significant associations

In the study to identify RLS risk factors, a GWA study was included in the analysis, in which all samples were of European ancestry. RLS status for cases was assessed either by face to face diagnosis or self-reported by means of a questionnaire. The RLS GWAS consisted of 6,228 cases and 10,992 controls. A subset of the samples had been used in previous RLS GWAS (Winkelmann, J. et al., Nat. Genet. 39, 1000-6 (2007); Schormair, B. et al., Nat. Genet. 40, 946-8 (2008); Winkelmann, J. et al., PLoS Genet. 7, e1002171 (2011)). Following study-specific quality control procedures and imputation using the 1000 Genomes Project reference panel, association analysis was carried out in each study using logistic regression on the gene dosages. Age, gender and multidimensional scaling (MDS) components were included as covariates. Detailed information on phenotyping, genotyping, quality control and association analysis of each of the two GWAS are provided in the Method section herein below.

After quality control, 7,080,534 single nucleotide polymorphisms (SNPs) and indels with a minor allele frequency (MAF) ≥ 1% were available for statistical analysis. A total of 13 independent association signals were identified with genome-wide significance. Independence was determined by assigning variants to clusters based on association P value, linkage disequilibrium (LD), and distance. LD structure was estimated using available individual-level genotype data of the RLS GWAS. Genomic control showed an inflation of the median test statistic (λ_{GC} = 1.16)), but LD Score regression indicated that 82.1 % of the inflation is accounted for by polygenicity (intercept = 1.04)).

### 1.2 Gene-centered description of associated loci

As strategy towards a biological interpretation of the associated risk loci, manual literature-based annotation for the protein-coding genes was performed, which i) contained associated variants or their LD-proxies, ii) were located in the LD-blocks tagged by the association signal (r² > 0.05), or iii), for loci containing no genes, were located in the vicinity of the LD-block (± 500kb from lead SNP and r² > 0.05).

Focusing on loci with only one candidate protein-coding gene, a list of seed genes for functional annotation was compiled: *SEMA6D* (15q21.1), *MYT1* (20q13.33), and the previously described genes *MEIS1* (2p14), *PTPRD* (9p24.1-p23), and *TOX3* (16q12.1).

SEMA6D is a class VI transmembrane semaphorin. These molecules are important in axonal pathfinding and signal through binding of receptor complexes composed of plexins, neuropilins and additional co-receptors (Pasterkamp, R. J., Nat. Rev. Neurosci. 13, 605-618 (2012).). SEMA6D has been shown to exert repulsive or attractive effects on axons, depending on the specific combinations of its main receptor Plexin A1 with coreceptors such as OTK or VEFFR2, respectively (Yoshida et al., Neuron 52, 775-788 (2006); Toyofuku, T. et al., Genes Dev. 18, 435-47 (2004)). *SEMA6D* knockout mice show misdirection of proprioceptive axons and their associated oligodendrocytes in the superficial dorsal horn, affecting proper synapse formation (Leslie, J. R. et al., Development 138, (2011)).

*MYT1* encodes a zinc-finger transcription factor expressed in neural progenitor cells in both central and peripheral nervous systems. It is involved in neuronal differentiation by suppressing neural progenitor fate and promoting neurogenesis (Vasconcelos, F. F. et al., Cell Rep. 17, 469-483 (2016)).

Both *MEIS1* and *TOX3* have been implicated in neurodevelopmental processes such as neurogenesis, neuronal cell type specification and establishing connectivity between neurons and their target field (Rataj-Baniowska, M. et al., J. Neurosci. 35, 14467-14475 (2015); Bouilloux, F. et al., Elife 5, (2016); Sahu, S. K. et al., Biochim. Biophys. Acta 1859, 833-40 (2016)).The association of *MEIS1* is the strongest validated RLS risk factor to date. For PTPRD, a function in axon guidance and synaptogenesis has been well established, especially for the formation of excitatory synapses (Takahashi, H. & Craig, A. M., Trends Neurosci. 36, 522-34 (2013); Li, Y. et al., Cell Rep. 12, 1618-30 (2015)).In addition, it was also shown to be involved in the initiation of axonal myelination by oligodendrocytes (Zhu, Q. et al., Neuroscience 308, 106-14 (2015)).

In the remaining 8 loci, several genes are located within the respective region or in its close proximity. Based on the annotation of the seed genes (see above), we highlight candidate genes in these loci, which have also been implicated in different aspects of neurodevelopment. In the chromosome 1p21.1 locus *NTNG1* encodes Netrin-G1, a presynaptic cell adhesion molecule involved in synapse formation (Zhang, Q. et al., Mol. Brain 9, 6 (2016)).It has been linked to several neurological and neuropsychiatric disorders such as atypical Rett syndrome and schizophrenia (Archer, H. L. et al., Am. J. Med. Genet. Part A 140A, 691-694 (2006); Wilcox, J. A. & Quadri, S., Psychiatr. Genet. 24, 266-268 (2014)). For the locus on chromosome 3p26, CRBN is a functional candidate. CRBN is the recognition component of a Cullin4a RING E3 ubiquitin ligase (Fischer, E. S. et al., Nature 512, 49-53 (2014)).It regulates the assembly and expression of calcium-activated potassium channels in brain regions involved in memory and learning (Rajadhyaksha, A. M. et al., Behav. Brain Res. 226, 428-434 (2012); Berkefeld, H. et al., Science (80-.). 314, (2006)). On chromosome 2q24.1, the association signal maps to two genes, *CCDC148* and *PKP4.* The latter encodes plakophilin 4 (p00071), a member of the armadillo protein family. It is a cell adhesion molecule which serves as a scaffold for signaling complexes and plays a role in cell adhesion and neurite outgrowth (Keil, R., Schulz, J. & Hatzfeld, M., Biol. Chem. 394, 1005-17 (2013)). Interestingly, PKP4 mediates Rab11-dependent recycling of proteins, including the transferrin receptor. This is of particular interest as alterations in iron metabolism have been consistently associated with RLS (Keil, R. & Hatzfeld, M., J. Cell Sci. 127, 60-71 (2014)). Another candidate in the region is TANC1, a synaptic protein which regulates growth of dendritic spines and excitatory synapses (Han, S. et al., J. Neurosci. 30, 15102-15112 (2010)).

Brain iron metabolism and the dopaminergic neurotransmitter system are current main suspects in explaining the pathophysiology of RLS. Apart from *PKP4* (iron metabolism).

### 1.3 Discussion

RLS presents as a combination of sensory and motor symptoms and consequently problems with initiating and maintaining sleep. The underlying neuronal pathways of somatosensory perception and motor behavior, and their integration, are controlled and modulated on different levels in the central as well as peripheral nervous system. This results in an abundance of opportunities for disease-causing events to happen. For RLS, the spatiotemporal dynamics of these events are still largely unclear. Functional analysis of RLS-associated variants in *MEIS1* pointed to an involvement of early developmental events in the ganglionic eminences, which form a transient structure during development and later on give rise to striatal and cortical cells (Spieler, D. et al., Genome Res. (2014). doi:10.1101/gr.166751.113). Furthermore, MEIS1 RNA and protein levels were found to be reduced in post-mortem brain tissue of risk-allele carriers of the RLS-associated SNP (Xiong, L. et al., Hum. Mol. Genet. 18, 1065-74 (2009)). Animal models of Meis1, Btbd9 and Ptprd recapitulated certain aspects of the RLS phenotype such as hyperactivity and sleep disturbances (Spieler, D. et al., Genome Res. (2014). doi:10.1101/gr.166751.113; Drgonova, J. et al., Mol. Med. 21, 717 (2015); DeAndrade, M. P. et al., Hum. Mol. Genet. 21, 3984-92 (2012)). Moreover, compounds such as illudalic acid analogs, which were shown to modify PTPRD activity, were highlighted as new targets for therapeutics (Earley, C. J., Uhl, G. R., Clemens, S. & Ferré, S., Sleep Med. (2016). doi:10.1016/j.sleep.2016.06.003).These results are a motivation to further exploit the GWAS approach to identify more loci, with the eventual goal of further driving understanding of the underlying biology and developing improved therapies. In the study performed, MEIS1 is confirmed as the strongest risk factor for RLS and a low-frequency SNP (rs113851554) is highlighted as a potentially causal regulatory variant of high impact (OR of 2.15). This undergirds MEIS1 as an excellent candidate for functional studies and animal models.

In order to identify candidate genes and pathways related to RLS, manual and bioinformatic (not shown herein) annotation approaches were applied. The results are highly overlapping and show a clear accumulation of genes related to different aspects of neurodevelopment. Several genes are implicated in neurogenesis and neuronal cell fate specification (*MYT1, MEIS1, TOX3*). Other genes cluster in functions related to cell-adhesion, axon guidance, and synapse formation (*PTPRD, NTNG1, SEMA6D).* Specifically, *PTPRD* and *NTNG1* were shown to be enriched in excitatory synapses (Loh, K. H. et al., Cell 166, 1295-1307.e21 (2016).).Adding support to the selection of these candidates is either evidence of known interaction between these genes or related proteins from the same family. MEIS1 can bind HOX proteins of all paralog groups and also participate in controlling HOX gene expression levels (Penkov, D. et al., Cell Rep. 3, 1321-1333 (2013); Williams, T. M., Williams, M. E. & Innis, J. W., Dev. Biol. 277, 457-471 (2005)). NTNG1 and its receptor NGL-1 were shown to interact with LAR, belonging to same family of receptor protein tyrosine phosphatases as PTPRD. This complex promoted synapse differentiation *in vitro* (Song, Y. S., Lee, H.-J., Prosselkov, P., Itohara, S. & Kim, E., J. Cell Sci. 126, 4926-4938 (2013)).BTBD9 is characterized by a presence of a BTB domain. Proteins with a BTB domain have been identified as the substrate recognizing unit of cullin 3 E3 ubiquitin ligases (Freeman, A. A. H., Mandilaras, K., Missirlis, F. & Sanyal, S., Fly (Austin). 7, 39-43 (2013)).

In conclusion, we identify 7 new risk loci for RLS, bringing the total number up to 13. The causal variants and their impact on gene function still have to be determined in order to validate genes as truly causal. Overall, candidate genes and pathways converge on functions important in the proper development of the central nervous system such as specification of neuronal identity and formation of neuronal circuitry (axon guidance and synaptogenesis), suggesting that perturbations in neurodevelopmental processes contribute to the risk of developing RLS. The results clearly support the previously posited concept of RLS as a neurodevelopmental disorder. The exact molecular mechanisms at play and the relevant time points are still unknown, but our identification of a new tranche of implicated loci, and the resulting candidate genes and pathways, provides a much-needed impetus for further functional RLS research.

### 1.4 Material and Methods

### Populations and study design

Participants of all studies provided informed consent and studies were approved by the respective ethical committees.

### RLS GWAS

The EU-RLS-GENE consortium GWAS includes samples from various countries with European heritage. In all RLS cases were recruited in specialized outpatient clinics for movement disorders. RLS diagnosis was based on a face-to-face interview by an expert neurologist, implementing the diagnostic criteria established by the IRLSSG in 2003. Population-matched controls were obtained for each case sample.

### RLS GWAS

All samples were genotyped on the Affymetrix Axiom CEU array. Genotyping was performed according to manufacturer's protocol. According to best practice recommendations by the manufacturer, genotype calling was performed in batches based on genotyping center and genotyping date using the Axiom GT1 algorithm.

Individuals were excluded if they had a callrate < 98%, had incorrect or ambiguous gender calls or showed potential sample contamination. We excluded related individuals (PIHAT ≥ 0.09375) and population outliers (deviation ≥ 4 SD from the population mean in MDS analysis). SNPs were excluded if they had a callrate < 98%, a MAF < 0.01, showed > 1 discordant genotype in duplicate samples, had a P-value for deviation from Hardy-Weinberg-Equilibrium ≤ 1x10⁻⁵ in controls or failed Affymetrix cluster quality criteria.

Imputation in the RLS GWAS was carried out in a two-step procedure. First, study genotypes were pre-phased to produce best-guess haplotypes. This was followed by imputation into these haplotypes with the 1000Genomes Phase 1 reference panel (Phase I integrated variant set release version 3). Pre-phasing was carried out per chromosome using SHAPEIT with standard settings, followed by imputation with IMPUTE2 in all samples in chunks of 5 MB with a buffer of 250kb.

Genetic association analysis was performed in SNPTEST v2.5.4. (Marchini, J., Howie, B., Myers, S., McVean, G. & Donnelly, P., Nat. Genet. 39, 906-913 (2007)) using an additive model and imputed dosages were used. Age, sex, and the first ten principal components from the MDS analysis in PLINK were included as covariates. SNPs with HWE p-value less than 10⁻⁵ and imputation score less than 0.5 were filter out.

### Example 2:

### 2.1 Patient

The 51-year-old female patient had been diagnosed with RLS at the age of 31 but had suffered from the disease since childhood. She had family history of RLS with her mother and two siblings being affected.

### 2.2 Protocol

The patient was washed off the concurrent medication, with the exception of two tablets of 0.18 mg pramipexole, which was maintained throughout the study.

After the washout period, the patient was observed for three weeks to establish a baseline level of her RLS symptoms and sleep disturbance. After the baseline period, she was prescribed 100 mg of the inhibitor of the cereblon (CRBN) thalidomide (i.e. 2-(2,6-dioxopiperidin-3-yl)-2,3-dihydro-1H-isoindole-1,3-dione) daily, which she continued taking for three weeks in addition to her two tables pramipexol 0,18 mg treatment.

Throughout the six-week period, the RLS severity and sleep disturbance of the patient were weekly observed with four different questionnaires:
1. IRLSSG severity scale: This is the gold standard questionnaire to evaluate RLS severity both in clinical practice and in pharmaceutical studies. The scale is based on 10 questions answered by the patient, and final severity score ranges from 0 to 40. On the IRLSSG scale, 0 points corresponds to no RLS, 1-10 points corresponds to mild RLS, 11-20 points corresponds to moderate RLS, 21-30 points corresponds to severe RLS and 31-40 points corresponds to very severe RLS.
2. RLS-6 scale. This is an alternative scale used to evaluate different aspects of RLS. The average score may be used to evaluate the overall severity of RLS. The scale ranges from 0 to 10, with 0 indicating no RLS and 10 being the most severe RLS severity score.
3. Epworth Sleepiness Scale (ESS). This is a gold standard questionnaire used to evaluate daytime sleepiness in clinical practice and in pharmaceutical studies. The scale ranges from 0 to 24, where 0-5 corresponds to normal daytime sleepiness, 6-10 corresponds to higher normal daytime sleepiness, 11-12 corresponds to mild excessive daytime sleepiness, 13-15 corresponds to moderate excessive daytime sleepiness and 16-24 corresponds to severe daytime sleepiness.
4. MOS Sleep Scale. This is a questionnaire used to evaluate sleep disturbance, sleep efficiency, sleep adequacy and other related parameters with different subscores derived from the answers. In this study, the sleep disturbance score was used. The range is 0-100%, 100% indicating the most severe sleep disturbance.

### 2.3 Results

The results of the IRLSSG severity scale, RLS-6 scale, Epworth Sleepiness Scale (ESS) and MOS sleep scale measurements are shown in Figures 1 to 4, respectively. The results are also summarized in the following Table 1:

**Table 1**

| **Week** | **Treatment** | **IRLSSG severity** | **RLS-6 score** | **ESS sleepiness** | **MOS sleep disturbance** |
|---|---|---|---|---|---|
| 1 | Baseline | 38 | 10 | 17 | 93.3 |
| 2 | Baseline | 36 | 8 | 15 | 46.7 |
| 3 | Baseline | 35 | 7.5 | 16 | 60 |
| 4 | 100 mg Thalidomide | 21 | 4.7 | 4 | 31.3 |
| 5 | 100 mg Thalidomide | 20 | 2.7 | 7 | 46.3 |
| 6 | 100 mg Thalidomide | 16 | 2 | 3 | 43.8 |
| 7 | Baseline | 22 | 7 | 14 | 72.5 |

As can be taken from the above-shown results, the RLS patient significantly benefitted from the treatment with the CRBN inhibitor thalidomide. A remarkable treatment success of RLS is evident on the basis of all four measured scores; see results of weeks 4-6.

## Claims

1. An inhibitor of the cereblon (CRBN) protein for use in the prevention or treatment of restless leg syndrome (RLS) and/or diseases associated therewith.

2. A method of preventing or treating restless leg syndrome (RLS) and/or diseases associated therewith comprising administering a pharmaceutically effective amount of an inhibitor of the cereblon (CRBN) protein to a subject in need thereof.

3. The inhibitor of claim 1 or the method of claim 2, wherein the inhibitor is an antibody or a fragment or derivative thereof, an aptamer, an siRNA, an shRNA, a miRNA, a ribozyme, an antisense nucleic acid molecule, a small molecule or modified versions of these inhibitors.

4. The inhibitor of claim 1 or 3, or the method of claim 2 or 3, wherein the inhibitor inhibits
(a) the binding of the cereblon (CRBN) protein to the ligand MEIS2;
(b) the binding of the cereblon (CRBN) protein to the damaged DNA binding protein 1 (DBB1);
(c) the binding of the cereblon (CRBN) protein to BK_{Ca} channels; and/or
(d) the binding of the cereblon (CRBN) protein to the voltage-gated chloride channel CIC-2.

5. The inhibitor of claim 4, or the method of claim 4, wherein the inhibitor inhibiting the binding of the cereblon (CRBN) protein to the ligand MEIS2 is selected from 2-(2,6-dioxopiperidin-3-yl)-2,3-dihydro-1H-isoindole-1,3-dione, (RS)-3-(4-Amino-1-oxo-1,3-dihydro-2H-isoindol-2-yl)piperidine-2,6-dione, (RS)-4-Amino-2-(2,6-dioxopiperidin-3-yl)isoindole-1,3-dione.

6. The inhibitor of any one of claims 1 or 3 to 5, or the method of any one of claims 2 to 5, wherein the diseases associated with RLS are depression, neuroticism, and/or periodic limb movement disorder.

7. A method of identifying an inhibitor of the cereblon (CRBN) protein suitable as a lead compound and/or as a medicament for the prevention and/or treatment of restless leg syndrome (RLS) and/or diseases associated therewith, comprising the steps of:
(a) determining the level of cereblon (CRBN) protein, the level of cereblon (CRBN) transcript and/or the level of activity of cereblon (CRBN) in a cell;
(b) contacting said cell or a cell of the same cell population with a test compound;
(c) determining the level of cereblon (CRBN) protein, the level of cereblon (CRBN) transcript and/or the level of activity of cereblon (CRBN) in said cell after contacting with the test compound; and
(d) comparing the level of cereblon (CRBN) protein, the level of cereblon (CRBN) transcript and/or the level of activity of cereblon (CRBN) determined in step (c) with the cereblon (CRBN) protein, the cereblon (CRBN) transcript and/or the cereblon (CRBN) activity level determined in step (a), wherein a decrease of cereblon (CRBN) protein, the cereblon (CRBN) transcript and/or the cereblon (CRBN) activity level in step (c) as compared to step (a) indicates that the test compound is an inhibitor of the cereblon (CRBN) protein suitable as a lead compound and/or as a medicament for the prevention and/or treatment of RLS and/or diseases associated therewith.

8. The method of claim 7, wherein the method further comprises a step (e) of validating the capability of the inhibitor identified in step (d) to serve as a lead compound and/or as a medicament for the prevention and/or treatment of restless leg syndrome (RLS).

9. The method of claim 7 or 8, further comprising optimising the pharmacological properties of an inhibitor of the cereblon (CRBN) protein identified as lead compound and/or as a medicament for the prevention and/or treatment of restless leg syndrome (RLS).

10. The method of claim 9, wherein the optimisation comprises modifying the inhibitor of cereblon (CRBN) protein to achieve:
i) modified spectrum of activity, organ specificity, and/or
ii) improved potency, and/or
iii) decreased toxicity (improved therapeutic index), and/or
iv) decreased side effects, and/or
v) modified onset of therapeutic action, duration of effect, and/or
vi) modified pharmacokinetic parameters (resorption, distribution, metabolism and excretion), and/or
vii) modified physico-chemical parameters (solubility, hygroscopicity, color, taste, odor, stability, state), and/or
viii) improved general specificity, organ/tissue specificity, and/or
ix) optimised application form and route
by
(a) esterification of carboxyl groups, or
(b) esterification of hydroxyl groups with carboxylic acids, or
(c) esterification of hydroxyl groups to, e.g. phosphates, pyrophosphates or sulfates or hemi-succinates, or
(d) formation of pharmaceutically acceptable salts, or
(e) formation of pharmaceutically acceptable complexes, or
(f) synthesis of pharmacologically active polymers, or
(g) introduction of hydrophilic moieties, or
(h) introduction/exchange of substituents on aromates or side chains, change of substituent pattern, or
(i) modification by introduction of isosteric or bioisosteric moieties, or
(j) synthesis of homologous compounds, or
(k) introduction of branched side chains, or
(l) conversion of alkyl substituents to cyclic analogues, or
(m) derivatisation of hydroxyl groups to ketales, acetales, or
(n) N-acetylation to amides, phenylcarbamates, or
(o) synthesis of Mannich bases, imines, or
(p) transformation of ketones or aldehydes to Schiffs bases, oximes, acetales, ketales, enolesters, oxazolidines, thiazolidines
(q) or combinations thereof.
